# EUROPEAN PATENT APPLICATION

(11) **EP 1 695 952 A1**
(43) Date of publication of application: **30.08.2006**
(21) Application number: 04820300.4
(22) Date of filing: 10.12.2004
(51) Int. Cl.: C07C 15/28, C09K 11/06, H05B 33/14

(54) **AROMATIC COMPOUND AND ORGANIC ELECTROLUMINESCENT DEVICE USING SAME**

(30) Priority: 15.12.2003 JP 2003417037
(71) Applicant: IDEMITSU KOSAN CO., LTD., Tokyo 100-8321 (JP)
(72) Inventor: IKEDA, Hidetsugu, Sodegaura-shi, Chiba 2990293 (JP); INOUE, Tetsuya, Sodegaura-shi, Chiba 2990293 (JP); KONDO, Hirofumi, Sodegaura-shi, Chiba 2990293 (JP)
(74) Representative: Gille Hrabal Struck Neidlein Prop Roos
(86) International application number: PCT/JP2004/018485
(87) International publication number: WO 2005/056505

(57) **Abstract**

An aromatic compound represented by a following general formula (1), wherein R¹ to R¹⁴ each independently represents any one selected from a group consisting of a hydrogen atom, a halogen atom, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted alkoxy group having 1 to 40 carbon atoms, a substituted or unsubstituted aryl group having 6 to 40 carbon atoms, a substituted or unsubstituted heteroaryl group having 3 to 40 carbon atoms; at least one of R¹ to R⁹ represents a substituted or unsubstituted aryl group having 6 to 40 carbon atoms; and at least one of R¹⁰ or R¹⁴ represents a substituted or unsubstituted aryl group having 6 to 40 carbon atoms. A compound for obtaining an organic EL device having an enhanced efficiency of light emission and a prolonged half lifetime of brightness is provided.

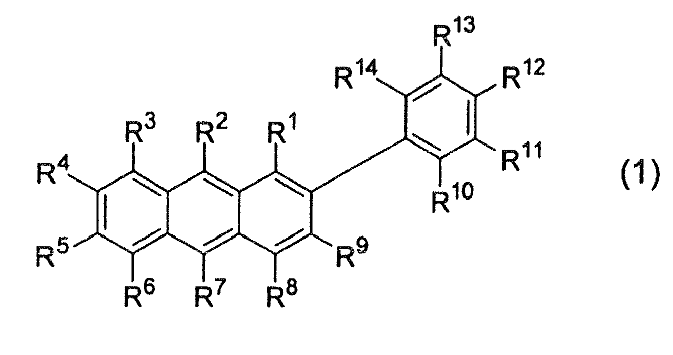

## Description

### TECHNICAL FIELD

The present invention relates to a novel aromatic compound and an organic electroluminescence device with a high efficiency of light emission and with a prolonged half lifetime of luminance. More particularly, the present invention relates to a novel aromatic compound with a specific structure, to an organic electroluminescence device using the aromatic compound, to a luminescent organic solution comprising the aromatic compound, and to an organic electroluminescence device which emits light by applying an electric field across a laminated thin film structure comprising the aromatic compound.

### BACKGROUND ART

In late years, display devices having advanced characteristics are demanded accompanied with an acceleration of development in information-communication industry. The display devices can be generally classified into light emission type display devices and non-light emission type display devices. The light emission type display devices include a cathode-ray tube, a light emitting diode (LED), etc. The non-light emission type display devices include a liquid crystal display, etc. As indices indicating basic characteristics of the display devices, there are operating voltage, power consumption, brightness, contrast, response time, lifetime, color representation, etc. The liquid crystal display device among the non-light emission type display devices is most broadly used nowadays because it is advantageous in light weight, consuming few powers. However, characteristics such as the response time, the contrast and a viewing angle did not arrived to a level of being enough yet, still remaining ample scope for improvement. Accordingly, an organic electroluminescence device (the word "electroluminescence" may be abbreviated to "EL" hereafter) attracts attention as the next generation display device which can supplement the above problems.
The organic EL device has advantages in not only with a wide viewing angle and a vivid contrast, but also with a fast response time all as a spontaneous emission type display device. The EL devices are classified into an inorganic EL device and an organic EL device dependent to its material for forming a light emitting layer. As compared with the inorganic EL device, the organic EL device has advantages in superiority of characteristics such as luminance, driving voltage, response speed and possibility in displaying multicolor. In general, an organic EL device has a constitution comprising a light emitting layer and a pair of electrodes sandwiching the light emitting layer. The light emission of the organic EL device is a phenomenon in which, when an electric field is applied between the two electrodes, electrons are injected from the cathode side and holes are injected from the anode side.
The electrons are recombined with the holes in the light emitting layer to form an excited state, and energy generated when the excited state returns to the ground state is emitted as light. Regarding with the material for forming the light emitting layer, an organic EL device employing a low molecular weight aromatic diamine and aluminum complex is reported (refer to, for example, Non-patent Literature 1 below). As the light emitting material of the organic EL device, coumarine derivatives, tetraphenylbutadiene derivatives, bisstyrylarylene derivatives and oxadiazole derivatives are known. It is reported that light in the visible region ranging from blue light to red light can be obtained by using these light emitting materials, and realization of a device exhibiting color images is expected (refer to, for example, Patent Literatures 1 to 3 below). Further, although an organic EL device employing an anthracene compound or its derivative is also proposed (refer to, for example, Patent literatures 4 to 13), an efficiency of light emission is so low in those technologies that more improvement in the efficiency of light emission is required.
For example, Patent Literature 4 below discloses an anthracene compound expressed by a following formula (a), and Patent Literature 8 below discloses an anthracene compound expressed by a following formula (b).

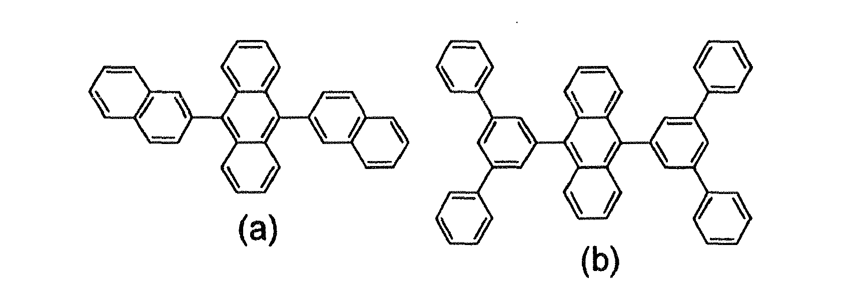

However, those anthracene compounds are extremely poor in solubility because 2-position is unsubstituted and accordingly, an enhancement of color purity by purification is difficult, an employment for wet-type film forming process is unable, and they are inferior in electroluminescent property.
A drawback of the anthracene compound exists in poor solubility owing to superiority in their flatness. Therefore, an anthracene compound having tert-butyl group or methyl group at 2-position of anthracene is proposed, and for example, Patent Literature 9 below discloses a compound expressed by a following formula (c), Patent Literatures 7 and 10 below disclose a compound expressed by a following formula (d), and Patent Literature 7 below discloses a compound expressed by a following formula (e).

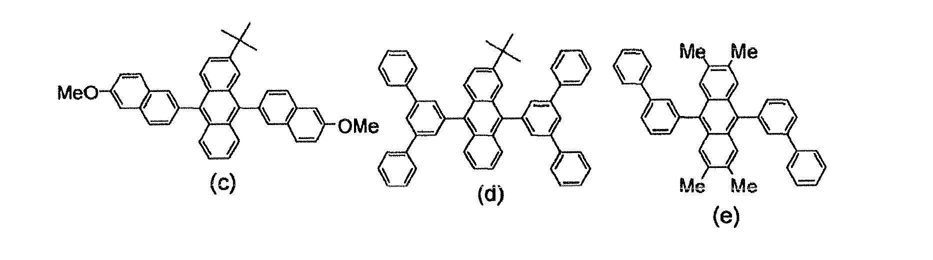

However, a steric hindrance of the level that the above anthracene compound has is incapable of making the compound soluble and as a result; the electroluminescent property is also inferior. Further, an anthracene compound having an aryl group at its side-chain is proposed, and for example, Patent Literature 5 below discloses a compound expressed by a following formula (f), Patent Literature 11 below discloses a compound expressed by a following formula (g), Patent Literature 12 below discloses a compound expressed by a following formula (h), and Patent Literature 13 below discloses a compound expressed by following formula (i).

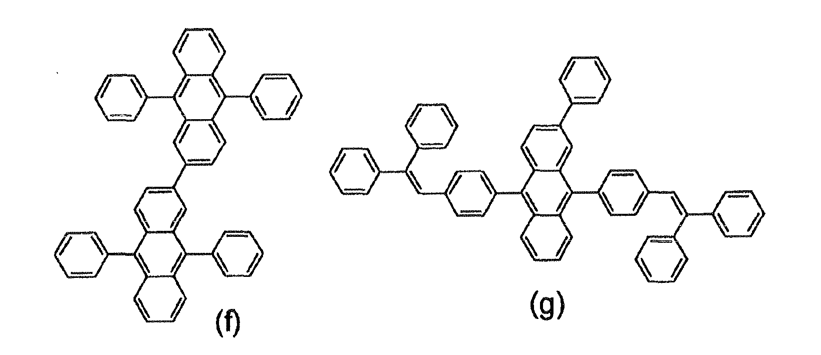

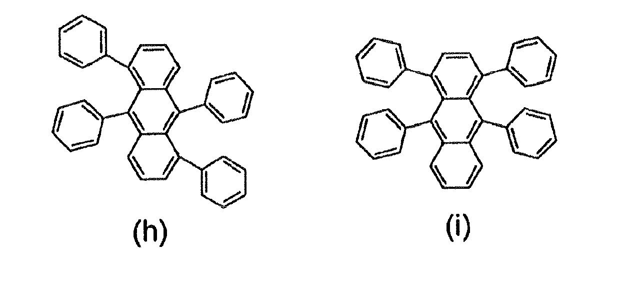

However, the above anthracene compounds have poor solubility owing to superiority in the flatness of the side-chain, and their electroluminescent properties are also not sufficient.
Besides, the organic electroluminescence device employing high polymer such as poly (p-phenylenevinylene) (PPV) or poly(2-methoxy-5-(2'-ethylhexyloxy)-1,4-phenylenevinylene) as the material for forming the light emitting layer is announced (refer to, for example, Non-patent Literature 2 below). Further, a soluble PPV was developed in which a functional group capable of improving a solubility characteristic into an organic solvent being introduced. A wet-type film forming of a solution containing the soluble PPV or its derivative in accordance with a process such as spin coating process, ink-jet process and so on enables to form the light emitting layer and as a result, to obtain the devices simply and easily. The organic electroluminescence device adopting PPV or its derivative as the material for the light emitting layer reveals light emission from green to orange.
On the other hand, the luminescent low molecular weight materials known at present are usually formed into the light emitting layer in accordance with a vacuum vapor deposition process because they have poor solubility in many cases. However, the vacuum vapor deposition process has many problems such that the process is complicated and that it requires large-scale vapor deposition apparatus. Accordingly, there was a demand for enabling to form the film for the devices simply and easily in accordance with the wet-type film forming as aforementioned even when a low molecular weight compound is employed. The luminescent low molecular weight compounds can be prepared simply and easily in accordance with a synthesizing route shorter than the above PPV and further, they have advantage in capability of being purified to an enhanced purity in accordance with a technology of publicly known column chromatography or so. Therefore, employing soluble low molecular weight compound was tried, however, it was practically unemployable alone because crystallization occurred after wet-type film formation caused pin-holes in a thin film and as a result, the soluble low molecular weight compound was formed to film in a state that it was dispersed into a binder resin, etc. However, because the binder resin is electrically inactive, there was a case of obstructing luminescent capability. As the above description, enabling to form a film of the soluble luminous compound in accordance with the wet-type film forming process, obtaining a light emitting layer of high quality and at the same time, enabling the devices comprising the soluble luminescent compound to have an enhanced efficiency of light emission are required. Further, because a lifetime of the devices of current levels is short, a large scale improvement is demanded.

Patent Literature 1: Japanese Unexamined Patent Application
   Laid-Open No. Hei 8(1996)-239655
Patent Literature 2: Japanese Unexamined Patent Application
   Laid-Open No. Hei 7(1995)-138561
Patent Literature 3: Japanese Unexamined Patent Application
   Laid-Open No. Hei 3(1991)-200289
Patent Literature 4: U. S. Patent No. 5,935,721
Patent Literature 5: Japanese Unexamined Patent Application
   Laid-Open No. Hei 8(1996)-012600
Patent Literature 6: Japanese Unexamined Patent Application
   Laid-Open No. 2000-344691
Patent Literature 7: Japanese Unexamined Patent Application
   Laid-Open No. Hei 11(1999)-323323
Patent Literature 8: U. S. Patent No. 5,972,247
Patent Literature 9: Japanese Unexamined Patent Application
   Laid-Open No. Hei 11(1999)-3782A
Patent Literature 10: Japanese Unexamined Patent Application
   Laid-Open No. Hei 11(1999)-329732
Patent Literature 11: Japanese Unexamined Patent Application
   Laid-Open No. 2001-335516
Patent Literature 12: Japanese Unexamined Patent Application
   Laid-Open No. Hei 10(1998)-294179
Patent Literature 13: U. S. Patent No. 5,077,142
Non-patent Literature 1: Appl.Phys.Lett.51, 913,1987
Non-patent Literature 2: Nature, 347,539,1990 &
   Appl.Phys.Lett.58, 1982,1991

### DISCLOSURE OF THE INVENTION

Taking the above circumstances into consideration, the present invention has an object of providing a compound for an organic EL device having an enhanced efficiency of light emission and a prolonged half lifetime of luminance. Further, the present invention has an object of providing a material for the organic EL device applicable not only to vapor deposition process but also to various kinds of wet-type film forming process.

As a result of intensive researches and zealous studies to achieve the above object by the present inventors, it was found that employing a novel aromatic compound containing an anthracene structure as a light emitting material enables to provide an organic EL device having an enhanced efficiency of light emission and prolonged half lifetime of luminance. Further, it was found that the novel aromatic compound exhibits excellent solubility for organic solvents revealing applicability for wet-type film forming process such as a spin coating process. Such being the case, the present invention has been accomplished on the basis of the foregoing findings and information.
Thus, the present invention provides an aromatic compound represented by a following general formula (1):

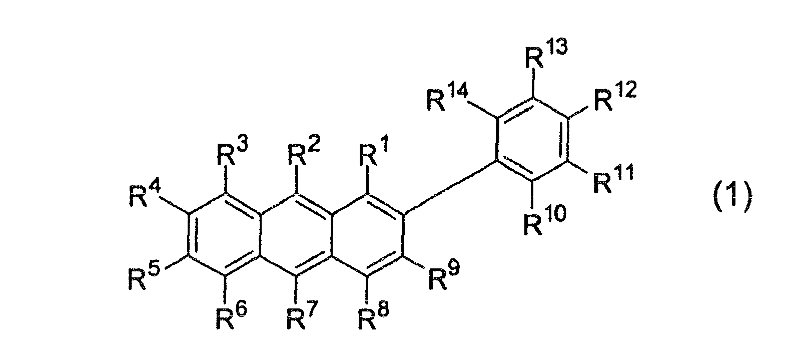

In the general formula (1), R¹ to R¹⁴ each independently represents any one selected from a group consisting of a hydrogen atom, a halogen atom, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted alkoxy group having 1 to 40 carbon atoms, a substituted or unsubstituted aryl group having 6 to 40 carbon atoms, a substituted or unsubstituted heteroaryl group having 3 to 40 carbon atoms. At least one of R¹ to R⁹ represents a substituted or unsubstituted aryl group having 6 to 40 carbon atoms, and R¹⁰ and/or R¹⁴ represents a substituted or unsubstituted aryl group having 6 to 40 carbon atoms.

Further, the present invention provides a luminescent organic solution which comprises the aromatic compound, and a material for an organic electroluminescence device which comprises the aromatic compound.
Furthermore, the present invention provides an organic electroluminescence device which comprises at least one organic thin film layer comprising a light emitting layer sandwiched between a pair of electrodes consisting of an anode and a cathode, wherein at least one of the organic thin film layer comprises the material for the organic electroluminescence device containing the above aromatic compound.

The present invention provides an organic EL device having an enhanced efficiency of light emission and a prolonged half lifetime of luminance. Further, the present invention provides a material for the organic EL device applicable not only for vapor deposition process but also for various kinds of wet-type film forming process.

### PREFERRED EMBODIMENT TO CARRY OUT THE INVENTION

The present invention provides a novel aromatic compound represented by the following general formula (1):

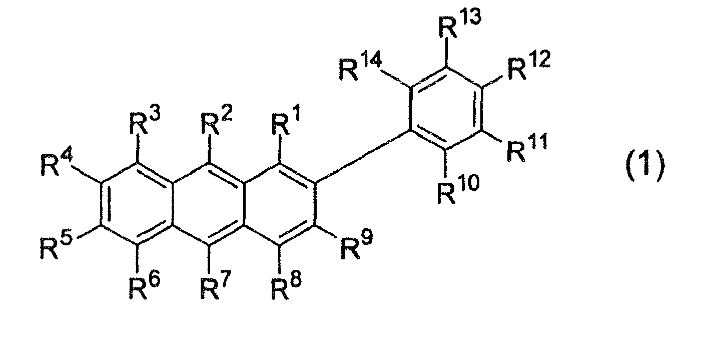

In the general formula (1), R¹ to R¹⁴ each independently represents any one selected from a group consisting of a hydrogen atom, a halogen atom, a substituted or unsubstituted alkyl group having 1 to 40, preferably 1 to 18 carbon atoms, a substituted or unsubstituted alkenyl group having 2 to 40, preferably 2 to 18 carbon atoms, a substituted or unsubstituted alkynyl group having 2 to 40, preferably 2 to 18 carbon atoms, a substituted or unsubstituted alkoxy group having 1 to 40, preferably 1 to 18 carbon atoms, a substituted or unsubstituted aryl group having 6 to 40, preferably 6 to 24 carbon atoms, and a substituted or unsubstituted heteroaryl group having 3 to 40, preferably 3 to 24 carbon atoms. At least one of R¹ to R⁹ represents a substituted or unsubstituted aryl group having 6 to 40, preferably 6 to 24 carbon atoms, and at least one of R¹⁰ or R¹⁴ represents a substituted or unsubstituted aryl group having 6 to 40, preferably 6 to 24 carbon atoms.
Examples of halogen atom include fluorine atom, chlorine atom, bromine atom and iodine atom.
Specific examples of the substituted or unsubstituted alkyl group having 1 to 40 carbon atoms include methyl group, ethyl group, 1-propyl group, 2-propyl group, 1-butyl group, 2-butyl group, sec-butyl group, tert-butyl group, pentyl group, hexyl group, octyl group, decyl group, dodecyl group, 2-ethylhexyl group, 3,7-dimethyl octyl group, cyclopropyl group, cyclopentyl group, cyclohexyl group, 1-adamanthyl group, 2-adamanthyl group, norbornyl group, trifluoromethyl group, trichloromethyl group, benzyl group, α, α -dimethylbenzyl group, 2-phenylethyl group, 1-phenylethyl group, etc

Specific examples of the substituted or unsubstituted alkenyl group having 2 to 40 carbon atoms include vinyl group, propenyl group, butenyl group, oleyl group, eicosapentaenyl group, docosahexaenyl group, 2,2-diphenylvinyl group, 1,2,2-triphenyl vinyl group, 2-phenyl-2-propenyl group, etc.
Specific examples of the substituted or unsubstituted alkynyl group having 2 to 40 carbon atoms include ethynyl group, methylethynyl group, phenylethynyl, etc.
Specific examples of the substituted or unsubstituted alkoxy group having 1 to 40 carbon atoms include methoxy group, ethoxy group, 1-propyloxy group, 2-propyloxy group, 1-butyloxy group, 2-butyloxy group, sec-butyloxy group, tert-butyloxy group, pentyloxy group, hexyloxy group, octyloxy group, decyloxy group, dodecyloxy group, 2-ethylhexyloxy group, 3,7-dimethyloctyloxy group, cyclopropyloxy group, cyclopentyloxy group, cyclohexyloxy group, 1-adamanthyloxy group, 2-adamanthyloxy group, norbornyloxy group, trifluoro methoxy group, benzyloxy group, α , α -dimethylbenzyloxy group, 2-phenylethoxy group, 1-phenylethoxy group, phenoxy group, etc.

Specific examples of the substituted or unsubstituted aryl group having 6 to 40 carbon atoms include phenyl group, 2-biphenylyl group, 3-biphenylyl group, 4-biphenylyl group, terphenylyl group, 3,5-diphenylphenyl group, 3,4-diphenylphenyl group, pentaphenylphenyl group, 4-(2,2-diphenylvinyl) phenyl group, 4-(1,2,2-triphenylvinyl)phenyl group, fluorenyl group, 1-naphthyl group, 2-naphthyl group, 9-anthryl group, 2-anthryl group, 9-phenanthryl group, 1-pyrenyl group, crycenyl group, naphthacenyl group, coronyl group, etc.
Specific examples of the substituted or unsubstituted heteroaryl group having 3 to 40 carbon atoms include a moiety or so of furan, thiophene, pyrrole, imidazole, pyrazole, triazole, oxadi azole, pyridine, pyrazine, triazine, benzofuran, dibenzofuran, benzothiophene, dibenzothiophene, carbazole, etc.

Preferable aromatic compound represented by the general formula (1) is a compound whose R¹ to R¹⁴ are selected from a group consisting of a hydrogen atom, an alkyl group having 1 to 40 carbon atoms, an aryl group having 6 to 40 carbon atoms, and a heteroaryl group having 3 to 40 carbon atoms. More preferable aromatic compound represented by the general formula (1) is a compound whose R¹ to R¹⁴ are selected from a group consisting of a hydrogen atom, an alkyl group having 1 to 40 carbon atoms, and an aryl group having 6 to 40 carbon atoms. Further more preferable aromatic compound represented by the general formula (1) is a compound whose R¹ to R¹⁴ are selected from a group consisting of a hydrogen atom and an aryl group having 6 to 40 carbon atoms. The best preferable aromatic compound represented by the general formula (1) is a compound whose R¹¹ to R¹³ are hydrogen atoms, any one of R¹⁰ or R¹⁴ is a phenyl group and another one is a hydrogen atom.
Specific examples of the aromatic compound represented by the general formula (1) of the present invention are as follows.

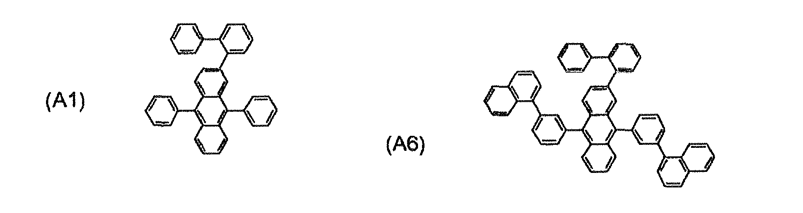

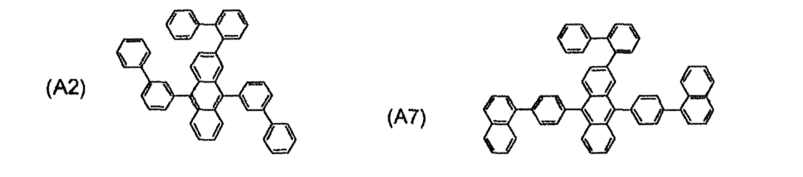

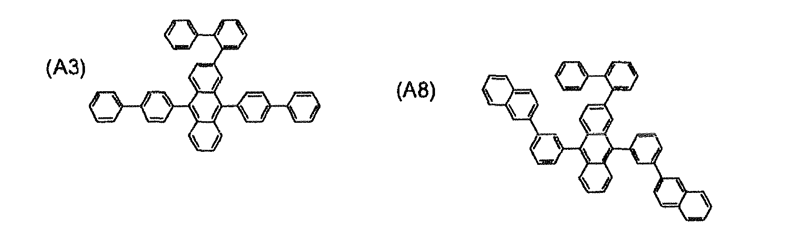

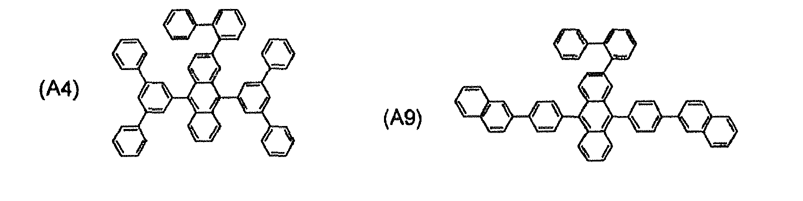

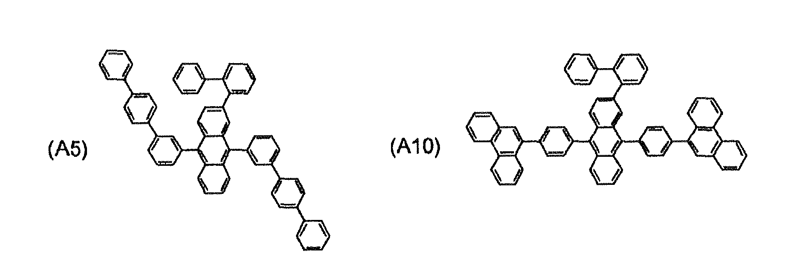

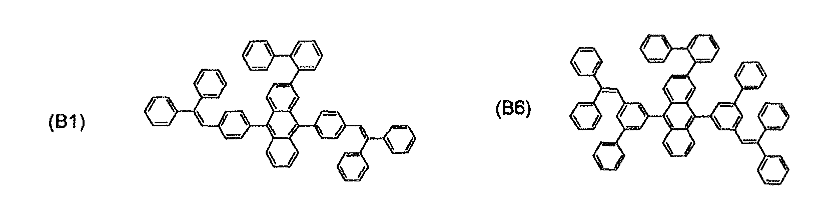

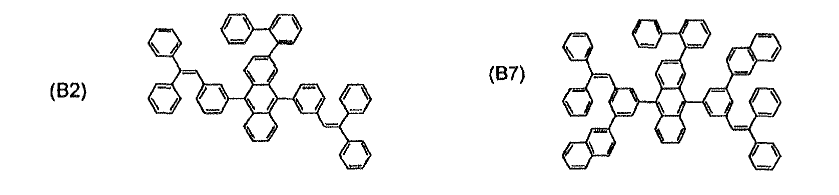

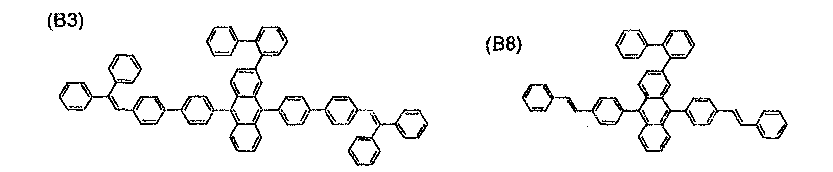

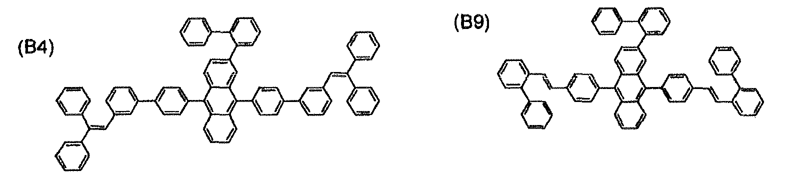

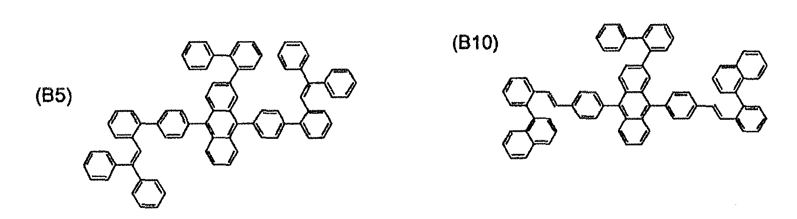

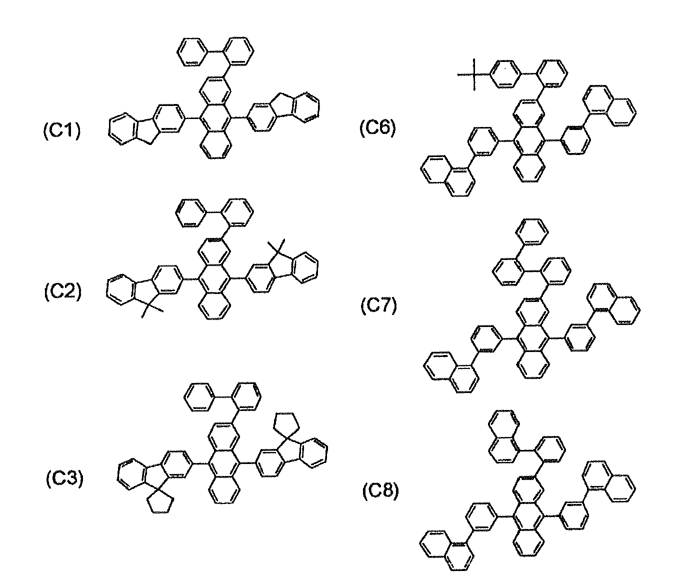

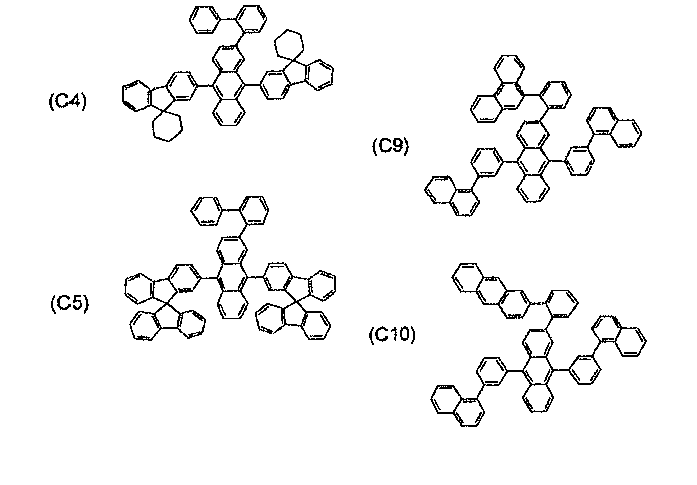

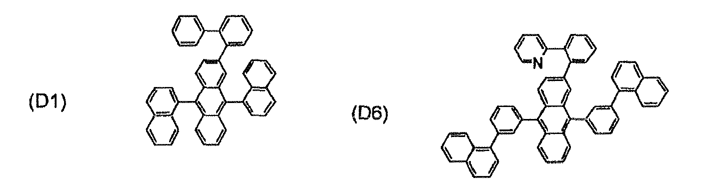

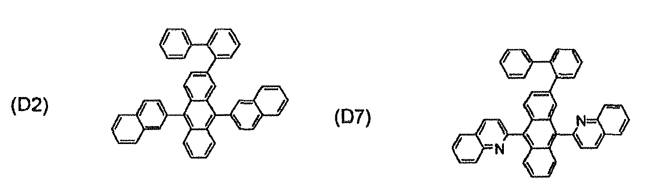

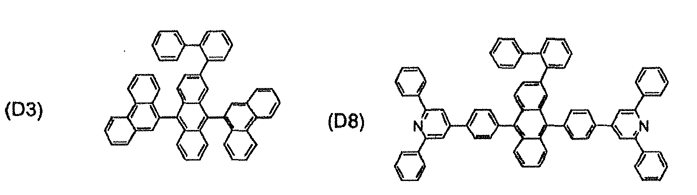

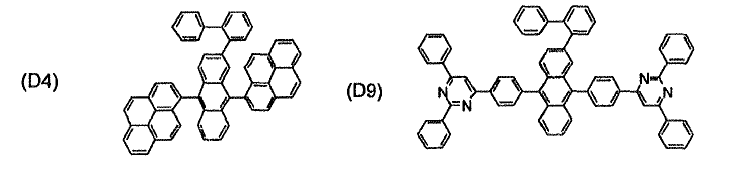

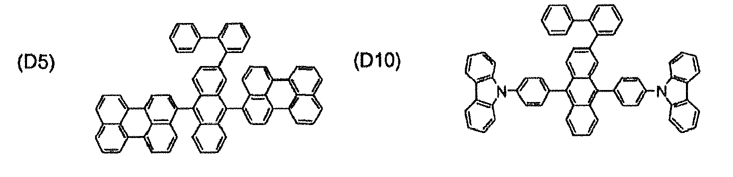

The device structure of the organic EL device of the present invention is a structure in which one or more organic layers are sandwiched between a pair of electrodes, and examples of the structure include (anode / light emitting layer /cathode) (anode / hole injecting or transporting layer / light emitting layer /electron injecting or transporting layer / cathode) (anode / hole injecting or transporting layer / light emitting layer / cathode), (anode / light emitting layer /electron injecting or transporting layer / cathode), etc. The aromatic compound in the present invention may be used in any of the foregoing organic layer, or may be doped into other hole transporting materials, light emitting materials and electron transporting materials. Further, the aromatic compound of the present invention may be used alone or may be used as a component of mixture.
The hole injecting or hole transporting material is preferably made of compounds which have a superior hole transporting ability as well as excellent capabilities of accepting holes injected from the anode and injecting the holes into the light emitting layer or light emitting material, prevent excited particles produced in the light emitting layer from moving into the electron injecting layer or electron injecting material, and exhibit an excellent capability of forming a thin film. Specific examples of the hole injecting or the hole transporting material include phthalocyanine derivatives, naphthalocyanine derivatives, porphyrin derivatives, oxazole, oxadiazole, triazole, imidazole, imidazolone, imidazolethione, pyrazoline, pyrazolone, tetrahydroimidazole, hydrazone, acylhydrazone, polyarylalkanes, stilbene, butadiene, benzidine-type triphenylamine, styryl amine-type triphenylamine, diamine-type triphenylamine and derivatives thereof, as well as high molecular materials such as polyvinylcarbazoles, polysilanes, and such typical high molecular conductive polymers as polyethylenedioxythiophene/polystyrene sulfonic acid (PEDOT/PSS), polyaniline/camphersulfonic acid (PANI/CSA) and so on though not particularly limited thereto.

Of those hole injecting materials usable in the organic EL device of the present invention, more effective hole injecting materials are aromatic tertiary amine derivatives and phthalocyanine derivatives. Specific examples of the aromatic tertiary amine derivatives include triphenyl amine, tritolyl amine, tolyldiphenyl amine, N,N'-diphenyl-N,N'-(3-methylphenyl)-1,1'-biphenyl-4,4'-diamine, N,N,N',N'-(4-methylphenyl)-1,1'-phenyl-4,4'-diamine, N,N,N',N'-(4-methylphenyl)-1,1'-biphenyl-4,4'-diamine, N,N'-diphenyl-N,N'-dinaphthyl-1,1'-biphenyl-4,4'-diamine, N,N'-(methylphenyl)-N,N'-(4-n-butylphenyl)-phenanthrene-9,10-diamine, N,N-bis(4-di-4-tolylaminophenyl)-4-phenyl-cylcohexane, and oligomers and polymers having these aromatic tertiary amine skeletons, though not particularly limited thereto.
Specific examples of the phthalocyanine (Pc) derivatives include phthalocyanine derivatives such as H₂P_{C}, CuPc, CoPc, NiPc, ZnPc, PdPc, FePc, MnPc, ClAlPc, ClGaPc, ClInPc, CISnPc, Cl₂SiPc, (HO)AlPc, (HO)GaPc, VOPc, TiOPc, MoOPc, GaPc-O-GaPc, as well as naphthalocyanine derivatives, though not particularly limited thereto.

The electron injecting or electron transporting material is preferably made of compounds which have a good electron transporting ability as well as excellent capabilities of accepting electrons injected from the cathode and injecting the electrons into the light emitting layer or light emitting material, prevent excited particles produced in the light emitting layer from moving into the hole injecting layer, and exhibit an excellent capability of forming a thin film. Specific examples of the electron injecting or electron transporting material include fluorenone, anthraquinodimethane, diphenoquinone, thiopyranedioxide, oxazole, oxadiazole, triazole, imidazole, perylenetetracarboxylic acid, fluorenylidenemethane, anthraquinodimethane, anthrone and derivatives thereof, though not particularly limited thereto. Further, an electron accepting substance and an electron donating substance may be added to the hole injecting material and the electron injecting material, respectively, for enhanced sensitization thereof.
In the organic EL device of the present invention, among those electron injecting materials, more effective electron injecting materials are metal complex compounds and nitrogen-containing five-membered ring derivatives. Specific examples of the metal complex compounds include 8-hydroxyquinolinato Lithium, bis(8-hydroxyquinolinato) zinc, bis(8-hydroxyquinolinato) copper, bis(8-hydroxyquinolinato) manganese, tris(8-hydroxyquinolinato) aluminum, tris(2-methyl-8-hydroxyquinolinato) aluminum, tris(8-hydroxyquinolinato) gallium, bis(10-hydroxybenzo[h]quinolinato) beryllium, bis(10-hydroxybenzo[h]quinolinato) zinc, bis(2-methyl-8-quinolinato) chlorogallium, bis(2-methyl-8-quinolinato) (o-cresolato) gallium, bis(2-methyl-8-quinolinato) (1-naphtholato) aluminum, and bis(2-methyl-8-quinolinato) (2-naphtholato) gallium, though not particularly limited thereto.

The nitrogen-containing five membered ring derivatives are preferably derivatives of oxazole, thiazole, oxadiazole, thiadiazole or triazole. Specific examples of the nitrogen-containing five membered ring derivatives include 2,5-bis(1-phenyl)-1,3,4-oxazole, dimethyl POPOP, 2,5-bis(1-phenyl)-1,3,4-thiazole, 2,5-bis(1-phenyl)-1,3,4-oxadiazole, 2-(4'-tert-butylphenyl)-5-(4"-biphenyl)-1,3,4-oxadiazole, 2,5-bis(1-naphthyl)-1,3,4-oxadiazole, 1,4-bis[2-(5-phenyloxadiazolyl)]benzene, 1,4-bis[2-(5-phenyloxadiazolyl)-4-tert-butylbenzene], 2-(4'-tert-butylphenyl)-5-(4"-biphenyl)-1,3,4-thiadiazole, 2,5-bis(1-naphthyl)-1,3,4-thiadiazole, 1,4-bis[2-(5-phenylthiadiazolyl)]benzene, 2-(4'-tert-butylphenyl)-5-(4"-biphenyl)-1,3,4-triazole, 2,5-bis(1-naphthyl)-1,3,4-triazole, and 1,4-bis[2-(5-phenyltriazolyl)]benzene, though not particularly limited thereto.

The electron injecting layer comprising the electric insulator or semiconductor enables to effectively prevent a leak of electric current and to improve the electron injection capability. It is preferable that at least one metal compound selected from the group consisting of alkali metal chalcogenides, alkaline earth metal chalcogenides, alkali metal halides and alkaline earth metal halides is used as the insulating material. It is preferable that the electron injecting layer is constituted with the above alkali metal chalcogenide since the electron injecting property can be improved. Preferable examples of the alliali metal halide include LiF, NaF, KF, LiCl, KCl and NaCl. Preferable examples of the alkaline earth metal halide include fluorides such as CaF₂, BaF₂, SrF₂, MgF₂ and BeF₂ and halides other than the fluorides. Preferable examples of the alkali metal halide include LiF, NaF, KF, LiCl, KCl and NaCl. Preferable examples of the alkaline earth metal halide include fluorides such as CaF₂, BaF₂, SrF₂, MgF₂ and BeF₂ and halides other than the fluorides.
Examples of the semiconductor constituting the electron transporting layer include oxides, nitrides and oxide nitrides containing at least one element selected from Ba, Ca, Sr, Yb, Al, Ga, In, Li, Na, Cd, Mg, Si, Ta, Sb and Zn, which are used singly or in combination of two or more. It is preferable that the inorganic compound constituting the electron transporting layer is in the form of a fine crystalline or amorphous insulating thin film. When the electron transporting layer is constituted with the above insulating thin film, a more uniform thin film can be formed and defective pixels such as dark spots can be decreased. Examples of the inorganic compound include the alkali metal chalcogenides, the alkaline earth metal chalcogenides, the alkali metal halides and the alkaline earth metal halides which are described above.

Further, the electron injecting layer may contain a reductive dorpant having work function of 2.9 eV or smaller. The reductive dopant used in the present invention is defined as a substance which reduces the electron transporting compound. Examples of the reductive dopant include at least one compound selected from alkali metals, alkali metallic complexes, alkali metal compounds, alkaline earth metals, alkaline earth metallic complexes, alkaline earth metal compounds, rare earth metals, rare earth metallic complexes and rare earth metal compounds. Examples of the alkali metal compound, the alkaline earth metal compound and the rare earth metal compound described above include oxides and halides of the respective metals. Examples of the preferable reductive dopant include at least one alkali metal selected from a group consisting of Li (the work function: 2.93 eV), Na (the work function: 2.36 eV), K (the work function: 2.28 eV), Rb (the work function: 2.16 eV) and Cs (the work function: 1.95 eV) or at least one alkaline earth metals selected from a group consisting of Ca (the work function: 2.9 eV), Sr (the work function: 2.0 to 2.5 eV) and Ba (the work function: 2.52 eV); whose work function of 3.0 eV or smaller is particularly preferable.
Among those, more preferable reductive dopants include at least one kind or more alkali metal selected from the group consisting of K, Rb and Cs, the latter Rb or Cs being farther more preferable and the last Cs being the most preferable. Those alkali metals have particularly high reducing capability, and only an addition of relatively small amount of them into an electron injection zone enables to achieve both improvement of luminance and lifetime extention of the organic EL device.

Further, with regard to the reductive dorpant with work function of 2.9 eV or smaller, a combination of two or more kinds of the alkali metal is also preferable, and particularly, combinations containing Cs, for example, combinations of Cs and Na, Cs and K, Cs and Rb, or Cs and Na and K are preferable. Containing Cs in combination enables to reveal reducing capability effectively, and the addition into the electron injection zone expects both improvement of luminance and lifetime extension of the organic EL device.
A fluorescent coloring agent may be doped into the light emitting layer. Although the concentration of doping is not particularly specified, preferable concentration is within a range of from 0.1 to 20 % by mass. Specific examples of the fluorescent coloring agent include publicly known fluorescent coloring agents such perylene derivative, rubrene derivative, coumarin derivative, dicyanomethylenepyran derivative, stilbene derivative, tristyrylamine derivative, distyrylarylene derivative, etc. Among those, preferable fluorescent coloring agent is distyrylarylene derivative, and the more preferable agent is arylamino substituted distyrylarylene derivative.

The anode of the organic EL device may be suitably made of a conductive material having a work function more than 4 eV. Examples of the conductive material for the anode include carbon, aluminum, vanadium, iron, cobalt, nickel, tungsten, silver, gold, platinum, palladium and alloys thereof, metal oxides such as tin oxide and indium oxide which are used for ITO substrates or NESA substrates, and organic conductive resins such as polythiophene and polypyrrole.
The cathode of the organic EL device according to the present invention may be suitably made of a conductive material having a work function of 4 eV or less. Examples of the electro-conductive material for the cathode include magnesium, calcium, tin, lead, titanium, yttrium, lithium, ruthenium, manganese, aluminum, lithium fluoride and alloys thereof, though not particularly limited thereto. Typical examples of the alloys include alloys of magnesium and silver, alloys of magnesium and indium, and alloys of lithium and aluminum, though not particularly limited thereto. The ratio between the constituting metals in the alloys may be controlled and appropriately determined depending upon temperature of vapor deposition sources, atmosphere, vacuum degree, etc. The anode and cathode may be constituted of two or more layers, if required.

At least one surface of the organic EL device of the present invention preferably exhibits a sufficient transparency in a wavelength range of light emitted therefrom in order to enhance an efficiency of light emission thereof. Further, the substrate for the device is also preferably transparent. The transparent electrode is formed using the above conductive material by vapor deposition method, sputtering method, etc., so as to ensure a desirable transparency thereof. The electrode disposed on a light emitting surface of the device preferably has a light transmittance of 10% or more. The substrate is not particularly limited as long as it suitably has a good mechanical and thermal strength as well as a good transparency. Examples of the substrate include glass substrates and transparent resin films.
Specific examples of the transparent resin films include films made of polyethylene, ethylene-vinyl acetate copolymer, ethylene-vinyl alcohol copolymer, polypropylene, polystyrene, polymethyl methacrylate, polyvinyl chloride, polyvinyl alcohol, polyvinyl butyral, nylons, polyether ether ketones, polysulfones, polyether sulfones, tetrafluoroethylene-perfluoroalkylvinyl ether copolymer, polyvinyl fluoride, tetrafluoroethylene-ethylene copolymer, tetrafluororethylene-hexafluoropropylene copolymer, polychlorotrifluoroethylene, polyvinylidene fluoride, polyesters, polycarbonates, polyurethanes, polyimides, and polyether imides, polyimide, polypropylene, etc. The respective layers of the organic EL device of the present invention may be formed by either a dry film-forming process such as vacuum deposition, sputtering, plasma and ion-plating, or a wet-type film-forming process such as spin-coating, dipping and flow-coating. The thickness of the respective layers is not particularly limited, but should be adjusted to an appropriate range. When the thickness is too large, a large electric voltage must be applied to the device in order to achieve a predetermined light output, resulting in a poor efficiency of light emission. On the other hand, when the thickness is too small, pinholes tend to be formed in the layers, thereby failing to obtain a sufficient luminance of light emitted even upon applying an electric field thereto.

In the wet-type film-forming process, materials constituting the respective layers are dissolved or dispersed in a suitable solvent to form a thin film thereof. Examples of the solvent include halogen based hydrocarbon solvent solvent such as dichloromethane, dichloroethane, chloroform, tetrachloromethane, tetrachloro ethane, trichloroethane, chlorobenzene, dichlorobenzene and chlorotoluene; ether based solvent such as dibutyl ether, tetrahydrofuran, dioxane and anisole; alcohol based solvent such as methanol, ethanol, propanol, butanol, pentanol, hexanol, cyclohexanol, methyl cellosolve, ethylcellosolve and ethylene glycol; hydrocarbon based solvent such as benzene, toluene, xylene, ethyl benzene, hexane, octane, decane and tetralin; and ester based solvent such as ethyl acetate, butyl acetate and amyl acetate. Among those, hydrocarbon based solvent such as toluene or dioxane and ether based solvent are preferable. Further, they may be used alone or in combination of two or more kinds thereof. Additionally, the employable solvent is not limited to the above examples.
The solvent used for forming the respective layers is not particularly limited. Also, suitable resins or additives may be added to the respective organic thin film layers for the purposes of improving a film-forming property, preventing formation of pinholes in the resultant film, etc. Examples of the resins usable for the above purposes include insulating resins such as polystyrene, polycarbonates, polyarylates, polyesters, polyamides, polyurethanes, polysulfones, polymethyl methacrylate, polymethyl acrylate and celluloses as well as copolymers thereof, photoconductive resins such as poly-N-vinyl carbazole and polysilanes, and conductive resins such as polythiophene and polypyrrole. Examples of the additives include antioxidants, ultraviolet absorbers and plasticizers.
To improve the stability of the organic EL of the present invention to heat, moisture and the atmosphere, a protective layer may be formed on the surface of the device or the entire device may be coated with a silicone oil or a resin for protection.

### EXAMPLE

This invention will be described in further detail with reference to Examples, which does not limit the scope of this invention.

### [Example 1]

Compound (A4) was synthesized in accordance with the following route of reactions:

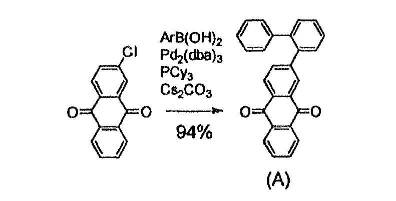

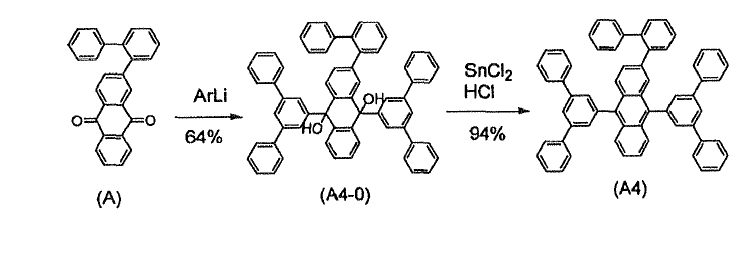

### (1) Synthesis of 2-(2-biphenylyl)-9,10-anthraquinone [Compound (A)]

Under an atmospheric argon gas flow, 2-chloroanthraquinone in an amount of 3.4 g (14 mmol), 2-biphenylylboronic acid in an amount of 5 g (17 mmol; 1.2 eq), tris(dibenzylideneacetone)dipalladium (0) in an amount of 0.32 g (0.35 mmol; 5 % Pd) and cesium carbonate in an amount of 14 g (43 mmol; 2.5 eq) are suspended into 40 milliliter of anhydrous dioxane, and adding a toluene solution of tricyclohexylphosphine in an amount 1.1 milliliter (25 % by mass, 0.98 mmol; 1.4 equivalent to Pd), the resultant solution was stirred at 80 °C for 10 hours.
The resultant reaction mixture was diluted with the use of water in an amount of 100 milliliter and further with the use of toluene in an amount of 300 milliliter and then, insolubles were filtrated by means of celite. An organic layer was separated from the filtrate and washed with the use of saturated sodium chloride solution in an amount of 50 milliliter and subsequently, dried with the use of sulfuric anhydride magnesium, followed by distillation of the solvent and as a result, deep red oil was obtained. The deep red oil was refined in accordance with column chromatography (filled with silicagel). The purification was carried out by eluting with the use of a mixed solvent of hexane and 33 % by mass of dichloromethane, followed by eluting with the use of mixed solvent of hexane and 50 % by mass of dichloromethane. As a result of the purification, 7.1 g of a pale brown solid compound was obtained (yield: 94 %). The pale brown solid was identified as the Compound (A) from the result in accordance with ¹H-NMR and Field Desorption Mass Spectrum (FD-MS) measurement. The result of the measurement in accordance with ¹H-NMR and FD-MS are shown as the following:
¹H-NMR (CDCl₃, TMS) δ 7.18(5H, s), 7.49 (5H, s), 7.76 (2H, dd, J=6Hz, 3Hz), 8.08 (1H, d, J=8Hz), 8.2-8.3 (3H, m)
FDMS, calcd for C₂₆H₁₆O₂=360, found m/z =360 (M⁺, 100)

### (2) Synthesis of 2-(2-biphenylyl)-9,10-bis(3,5-diphenylphenyl)-9,10-dihydroxy -9,10-dihydro anthracene [Compound (A4-0)]

Under an atmospheric argon gas flow, 3,5-diphenyl-1-bromobenzene in an amount of 3.9 g (13 mmol, 3eq) was dissolved into a mixed solvent of anhydrous toluene in an amount of 20 milliliter and anhydrous THF (tetrahydrofuran) in an amount of 20 milliliter, followed by cooling down to a temperature of -20 °C by means of dry ice / methanol bath. Adding hexane solution of n-butyllithium in an amount of 8.6 milliliter (1.59 mol/L, 14 mmol; 1.05 eq), the resultant solution was stirred at the temperature of ―20 °C for 1 hour. Into the resultant solution, 2-(2-biphenylyl)-9,10-anthraquinone [Compound (A)] in an amount of 1.5 g (4.2 mmol) was added and after stirring at a room temperature for 5 hours, it was stood alone for a night.
The resultant reaction mixture was deactivated with the use of saturated ammonium chloride aqueous solution in an amount of 50 milliliter, and an organic layer was separated. The organic layer was washed with the use of saturated sodium chloride solution in an amount of 50 milliliter and then, dried with the use of magnesium sulfate, followed by distillation of the solvent and as a result, red oil was obtained. The red oil was purified in accordance with column chromatography (filled with silicagel). The purification was carried out by eluting with the use of a mixed solvent of hexane and 50 % by mass of dichloro-methane, followed by eluting with the use of mixed solvent of dichloro-methane and 3 % by mass of methanol. As a result of the purification, 2.2 g of a white solid compound was obtained (yield: 30 %). The white solid was identified as the Compound (A4-0) in accordance with ¹H-NMR. The result of the measurement in accordance with ¹H-NMR is shown as the following:
¹H-NMR (CDCl₃, TMS) δ 2.53 (1H, s), 2.86 (1H, s), 6.8-6.9 (3H, m), 7.0-7.3 (27H, m), 7.4-7.5 (7H, m), 7.77 (2H, s), 7.9-8.0 (3H, m)

### (3) Synthesis of 2-(2-biphenylyl)-9,10-bis (3,5-diphenylphenyl)anthracene [Compound (A4)]

Suspending 2-(2-biphenylyl)-9,10-bis(3,5-diphenylphenyl)-9,10-dihydroxy-9,10-dihydro anthracene [Compound (A4-0)] in an amount of 2.2 g (2.7mmol) and stannic chloride dihydrate in an amount of 12 g (53 mmol; 20eq) into 45 milliliter of THF, and adding concentrated hydrochloric acid in an amount of 28 milliliter, the resultant solution was refluxed for 10 hours.
The resultant reaction mixture was separated by filtration, washed and dried with the use of water and methanol, and as a result, pale yellow solid was obtained. The pale yellow solid was purified in accordance with column chromatography (filled with silicagel). The purification was carried out by eluting with the use of a mixed solvent of hexane and 33 % by mass of dichloromethane, followed by eluting with the use of dichloromethane. As a result of the purification, 2.0 g of a pale yellow solid compound was obtained (yield: 94 %). The pale yellow solid was identified as the Compound (A4) from the results in accordance with ¹H-NMR and FD-MS measurement, etc. The result of the measurement in accordance with ¹H-NMR and FD-MS are shown as the following. Additionally, Ip means an ionization potential, Eg means an energy gap and Tg means a glass transition temperature (the same as in the following description).

¹H-NMR (CDCl₃, TMS) δ 6.7-6.9 (5H, m), 7.2-7.8 (35H, m), 7.95 (1H, s), 8.02 (1H, s)
FDMS, calcd for C₆₂H₄₂=786, found m/z =786 (M⁺, 100)
λ max, 406,385,366 nm (PhMe)
Fmax, 426,445 nm (PhMe, λ ex =406 nm)
Ip =5.75 eV (100 nanowatt, 31 Y/eV)
Eg =2.95 eV
Tg= 149 °C
Solubility of toluene: 40 mg/milliliter

### [Example 2]

Compound (A5) was synthesized in accordance with the following route of reactions:

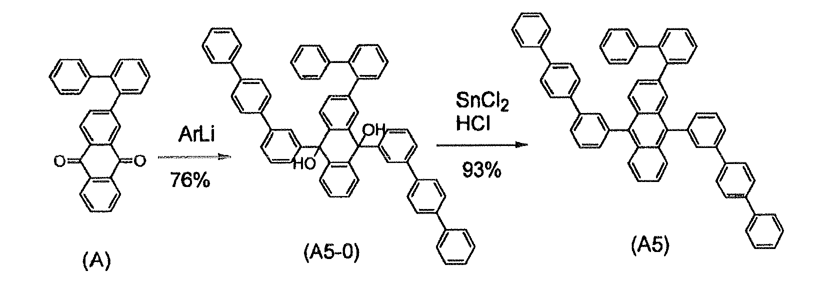

### (1) Synthesis of 2-(2-biphenylyl)-9,10-bis(3-(4-biphenylyl)phenyl)-9,10-dihydroxy-9,10-dihydro anthracene [Compound (A5-0)]

Under an atmospheric argon gas flow, 3-(4-biphenylyl)-1-bromobenzene in an amount of 5.9 g (19 mmol, 3eq) was dissolved into a mixed solvent of anhydrous toluene in an amount of 90 milliliter and anhydrous THF in an amount of 40 milliliter, followed by cooling down to a temperature of -20 °C by means of dry ice / methanol bath. Adding hexane solution of n-butyllithium in an amount of 13 milliliter (1.59 mol/litter, 1.21 mmol; 1.1 eq), the resultant solution was stirred at a temperature of ―20 °C for 1 hour. Into the resultant solution, 2-(2-biphenylyl)-9,10-anthraquinone [Compound (A)] in an amount of 2.3 g (6.4 mmol) was added and after stirring at a room temperature for 9 hours, it was stood alone for a night.
The resultant reaction mixture was deactivated with the use of saturated ammonium chloride aqueous solution in an amount of 50 milliliter, and an organic layer was separated. The organic layer was washed with the use of saturated sodium chloride solution in an amount of 50 milliliter and then, dried with the use of magnesium sulfate, followed by distillation of the solvent and as a result, a pale yellow solid was obtained. The pale yellow solid was washed with the use of a mixed solvent of hexane : dichloromethane =1:1 (capacity ratio) and as a result, 4.0 g of white solid compound was obtained (yield: 76 %). The white solid was identified as the Compound (A5-0) in accordance with ¹H-NMR. The measurement results of ¹H-NMR are shown as the following:
¹H-NMR (CDCl₃, TMS) δ 2.38 (1H, s), 2.84 (1H, s), 6.9-7.8 (42 H, m)

### (2) Synthesis of 2-(2-biphenylyl)-9,10-bis(3-(4-biphenylyl)phenyl)anthracene [Compound (A5)]

Suspending 2-(2-biphenylyl)-9,10-bis(3,5-diphenylphenyl)-9,10-dihydroxy-9,10-dihydro anthracene [Compound (A5-0)] in an amount of 4.0 g (4.9 mmol) and stannic chloride dehydrate in an amount of 22 g (97 mmol; 20eq) into 100 milliliter of THF, and adding concentrated hydrochloric acid in an amount of 50 milliliter, the resultant solution was refluxed for 10 hours.
The resultant reaction mixture was separated by filtration, washed with the use of water and methanol successively, followed by and drying and as a result, pale yellow solid was obtained. The pale yellow sold was purified in accordance with column chromatography (filled with silicagel). The purification was carried out by eluting with the use of a mixed solvent of hexane and 20 % by mass of dichloromethane, followed by eluting with the use of dichloromethane. As a result of the purification, 3.6 g of a pale yellow solid compound was obtained (yield: 93 %). The pale yellow solid was identified as the Compound (A5) from the results in accordance with ¹H-NMR and FD-MS measurement, etc. The results of the measurement in accordance with ¹H-NMR and FD-MS are shown as the following:

¹H-NMR (CDCl₃, TMS) δ 7.03 (5H, s), 7.3-7.8 (37H, m)
FDMS, calcd for C₆₂H₄₂=786, found m/z =786 (M⁺, 100)
λ max, 405,385,366 nm (PhMe)
Fmax, 424,445 nm (PhMe, λ ex =400 nm)
Ip= 5.76 eV (100 nanowatt, 31Y/eV)
Eg= 2.94 eV
Tg= 148 °C
Solubility of toluene: 110 mg/milliliter or greater

### [Example 3]

Compound (A6) was synthesized in accordance with the following route of reactions:

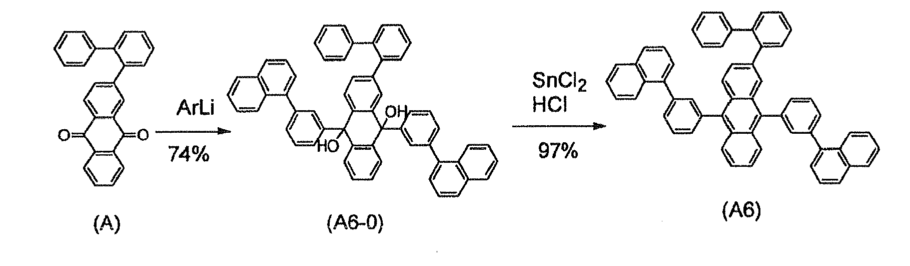

### (1) Synthesis of 2-(2-biphenylyl)-9,10-bis(3-(1-naphthyl)phenyl)-9,10-dihydroxy-9,10-dihydro anthracene [Compound (A6-0)]

Under an atmospheric argon gas flow, 3-(4-biphenylyl)-1-bromobenzene in an amount of 4.2 g (15 mmol; 2.7 eq) was dissolved into a mixed solvent of anhydrous toluene in an amount of 25 milliliter and anhydrous THF in an amount of 25 milliliter, followed by cooling down to a temperature of ―20 °C by means of dry ice / methanol bath. Adding hexane solution of n-butyllithium in an amount of 10 milliliter (1.59 mol/litter, 15.9 mmol; 1.06 eq), the resultant solution was stirred at the temperature of -20 °C for 1 hour. Into the resultant solution, 2-(2-biphenylyl)-9,10-anthraquinone [Compound (A)] in an amount of 2.0 g (5.6 mmol) was added and after stirring at the room temperature for 5 hours, it was stood alone for a night.
The resultant reaction mixture was deactivated with the use of saturated ammonium chloride aqueous solution in an amount of 50 milliliter, and an organic layer was separated. The organic layer was washed with the use of saturated sodium chloride solution in an amount of 50 milliliter and then, dried with the use of magnesium sulfate, followed by distillation of the solvent and as a result, an yellow oil was obtained. The yellow oil was purified in accordance with column chromatography (filled with silicagel). The purification was carried out by eluting with the use of a mixed solvent of hexane and 50 % by mass of dichloro-methane, followed by eluting with the use of mixed solvent of dichloro-methane and 3 % by mass of methanol. As a result of the purification, 3.1 g of a pale yellow amorphous solid compound was obtained (yield: 74 %). The white solid was identified as the Compound (A6-0) in accordance with ¹H-NMR. The measurement results of ¹H-NMR are shown as the following:
¹H-NMR (CDCl₃, TMS) δ 2.36 (1H, s), 2.89 (1H, s), 6.7-7.9 (38H, m)

### (2) Synthesis of 2-(2-biphenylyl)-9,10-bis(3-(1-naphthyl)phenyl)anthracene [Compound (A6)]

Suspending 2-(2-biphenylyl)-9,10-bis(3,5-diphenylphenyl)-9,10-dihydroxy-9,10-dihydro anthracene [Compound (A6-0)] in an amount of 1.1 g (2.7mmol) and stannic chloride dehydrate in an amount of 6.5 g (29 mmol; 20 eq) into 25 milliliter of THF, and adding concentrated hydrochloric acid in an amount of 25 milliliter, the resultant solution was refluxed for 10 hours.
The resultant reaction mixture was separated by filtration, washed with the use of water and methanol successively, followed by drying and as a result, pale yellow solid was obtained. The pale yellow solid was purified in accordance with column chromatography (filled with silicagel). The purification was carried out by eluting with the use of a mixed solvent of hexane and 20 % by mass of dichloromethane. As a result of the purification, 1.0 g of a pale yellow solid was obtained (yield: 97 %). The pale yellow solid was identified as the Compound (A6) from the results in accordance with ¹H-NMR and FD-MS measurement, etc. The results of the measurement in accordance with ¹H-NMR and FD-MS are shown as the following:

¹H-NMR (CDCl₃, TMS) δ 6.93 (5H, bs), 7.2-7.9 (31H, m), 8.0-8.1 (2H, m)
FDMS, calcd for C₅₈H₃₈= 734, found m/z =734 (M⁺, 100)
λ max, 407,385,366 nm (PhMe)
Fmax, 426,446 nm (PhMe, λ ex =405 nm)
Ip= 5.69 eV (500 nanowatt, 82Y/eV)
Eg= 2.92 eV
Tg= 130 °C
Solubility of toluene: 102 mg/milliliter or greater

### [Example 4]

Compound (A7) was synthesized in accordance with the following route of reactions:

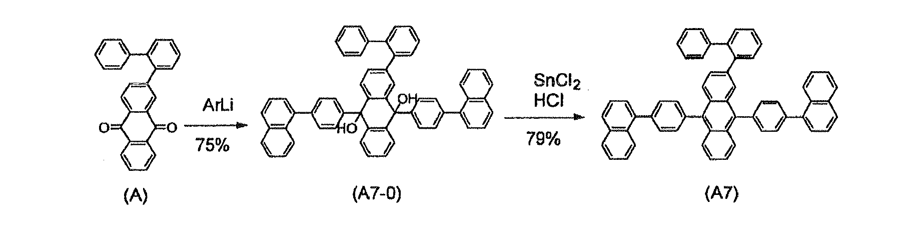

### (1) Synthesis of 2-(2-biphenylyl)-9,10-bis(4-(1-naphthyl)phenyl)-9,10-dihydroxy-9,10-dihydro anthracene [Compound (A7-0)]

Under an atmospheric argon gas flow, 4-(1-naphthyl)-1-bromobenzene in an amount of 4.2 g (15 mmol, 2.7 eq) was dissolved into a mixed solvent of anhydrous toluene in an amount of 25 milliliter and anhydrous THF in an amount of 25 milliliter, followed by cooling down to a temperature of ―20 °C by means of dry ice / methanol bath. Adding hexane solution of n-butyllithium in an amount of 10 milliliter (1.59 mol/litter, 15.9 mmol; 1.06 eq), the resultant solution was stirred at the temperature of -20 °C for 1 hour. Into the resultant solution, 2-(2-biphenylyl)-9,10-anthraquinone [Compound (A)] in an amount of 2.0 g (5.6 mmol) was added and after stirring at a room temperature for 2 hours, it was stood alone for a night.
The resultant reaction mixture was deactivated with the use of saturated ammonium chloride aqueous solution in an amount of 50 milliliter, and an organic layer was separated. The organic layer was washed with the use of saturated sodium chloride solution in an amount of 50 milliliter and then, dried with the use of magnesium sulfate, followed by distillation of the solvent and as a result, yellow oil was obtained. The yellow oil was purified in accordance with column chromatography (filled with silicagel). The purification was carried out by eluting with the use of a mixed solvent of hexane and 50 % by mass of dichloromethane, followed by eluting with the use of mixed solvent of dichloromethane and 3 % by mass of methanol. As a result of the purification, 3.1 g of a pale yellow amorphous solid compound was obtained (yield: 75 %). The pale yellow amorphous solid was identified as the Compound (A7-0) in accordance with ¹H-NMR. The measurement results of ¹H-NMR are shown as the following:
¹H-NMR (CDCl₃, TMS) δ 2.36 (1H, s), 2.89 (1H, s), 6.7-7.9 (38H, m)

### (2) Synthesis of 2-(2-biphenylyl)-9,10-bis(4-(1-naphthyl)phenyl)anthracene [Compound (A7)]

Suspending 2-(2-biphenylyl)-9,10-bis(4-(1-naphtyl)phenyl)-9, 10-dihydroxy-9,10-dihydro anthracene [Compound (A7-0)] in an amount of 1.1 g (1.4 mmol) and stannic chloride dehydrate in an amount of 6.5 g (29 mmol; 20eq) into 25 milliliter of THF, and adding concentrated hydrochloric acid in an amount of 15 milliliter, the resultant solution was refluxed for 10 hours.
The resultant reaction mixture was separated by filtration, washed with the use of water and methanol successively, followed by drying and as a result, pale yellow solid was obtained. The pale yellow solid was purified in accordance with column chromatography (filled with silicagel). The purification was carried out by eluting with the use of a mixed solvent of hexane and 20 % by mass of dichloromethane. As a result of the purification, 1.0 g of a pale yellow solid compound was obtained (yield: 79 %). The pale yellow solid was identified as the Compound (A7) from the results in accordance with ¹H-NMR and FD-MS measurement, etc. The results of the measurement in accordance with ¹H-NMR and FD-MS are shown as the following:

¹H-NMR (CDCl₃, TMS) δ 6.93 (5H, bs), 7.2-7.9 (31H, m), 8.0-8.1 (2H, m)
FDM S, calcd for C₅₈H₃₈= 734, found m/z =734 (M⁺, 100)
λ max, 408,385,366 nm (PhMe)
Fmax, 427,446 nm (PhMe, λ ex =405 nm)
Ip =5.70 eV (100 nanowatt, 19Y/eV)
Eg =2.92 eV
Tg= 146 °C
Solubility of toluene: 100 mg/milliliter or greater

### [Example 5]

Compound (B1) was synthesized in accordance with the following route of reactions:

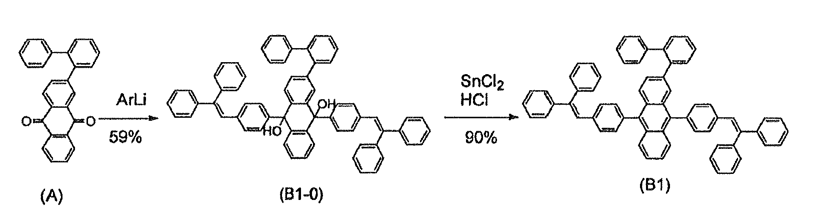

### (1) Synthesis of 2-(2-biphenylyl)-9,10-bis(4-(2,2-diphenylvinyl)phenyl)-9,10-dihydroxy-9,10-dihydro anthracene [Compound (B1-0)]

Under an atmospheric argon gas flow, 4-(2,2-diphenylvinyl)-1-bromobenzene in an amount of 9.8 g (29 mmol, 3 eq) was dissolved into a mixed solvent of anhydrous toluene in an amount of 45 milliliter and anhydrous THF in an amount of 45 milliliter, followed by cooling down to the temperature of ― 20 °C by means of dry ice / methanol bath. Adding hexane solution of n-butyllithium in an amount of 19 milliliter (1.59 mol/litter, 30 mmol; 1.04 eq), the resultant solution was stirred at the temperature of -20 °C for 1 hour. Into the resultant solution, 2-(2-biphenylyl)-9,10-anthraquinone [Compound (A)] in an amount of 3.5 g (9.7 mmol) was added and after stirring at the room temperature for 2 hours, it was stood alone for a night.
The resultant reaction mixture was deactivated with the use of saturated ammonium chloride aqueous solution in an amount of 50 milliliter, and the resultant solid was separated. The solid was washed use of the mixed solvent of hexane : dichloromethane = 1:1 (capacity ratio) and as a result, 5.0 g of white solid compound was obtained (yield: 59 %). The white solid was identified as the Compound (B1-0) in accordance with ¹H-NMR. The measurement results of
¹ H-NMR are shown as the following:
¹H-NMR (CDCl₃, TMS) δ 2.17 (1H, s), 2.66 (1H, s), 6.21 (2H, d, J=8Hz), 6.51 (2H, d, J=8Hz), 6.55 (4H, s), 6.80 (2H, d, J=4Hz), 7.0-7.4 (3H, m), 7.6-7.7 (3H, m)

### (2) Synthesis of 2-(2-biphenylyl)-9,10-bis(4-(2,2-diphenylvinyl)phenyl) anthracene [Compound (B1)]

Suspending 2-(2-biphenylyl)-9, 10-bis(4-(2,2-diphenylvinyl)-9,10-dihydroxy-9,10-dihydro anthracene [Compound (B1-0)] in an amount of 5.0 g (5.7 mmol) and stannic chloride dihydrate in an amount of 26 g (0.12 mol; 20eq) into 100 milliliter of THF, and adding concentrated hydrochloric acid in an amount of 60 milliliter, the resultant solution was refluxed for 10 hours.
The resultant reaction mixture was separated by filtration, washed with the use of water and methanol successively, followed by drying and as a result, pale yellow solid was obtained. The pale yellow solid was purified in accordance with column chromatography (filled with silicagel). The purification was carried out by eluting with the use of a mixed solvent of hexane and 33 % by mass of dichloromethane. As a result of the purification, 4.3 g of a pale yellow solid compound was obtained (yield: 90 %). The pale yellow solid was identified as the Compound (B1) from the results in accordance with ¹H-NMR and FD-MS measurement, etc. The results of the measurement in accordance with ¹H-NMR and FD-MS are shown as the following:

¹H-NMR (CDCl₃, TMS) δ 6.83 (2H, d, J=8Hz), 7.0-7.4 (40H, m), 7.5-7.7 (4H, m)
FDMS, calcd for C₆₆H₄₆= 838, found m/z =838 (M⁺, 100)
λ max, 408,390 nm (PhMe)
Fmax, 453 nm (PhMe, λ ex =400 nm)
Ip =5.65 eV (100 nanowatt, 75Y/eV)
Eg =2.85 eV
Tg=120 °C
Solubility of toluene: 104 mg/milliliter or greater

### [Example 6]

Compound (B5) was synthesized in accordance with the following route of reactions:

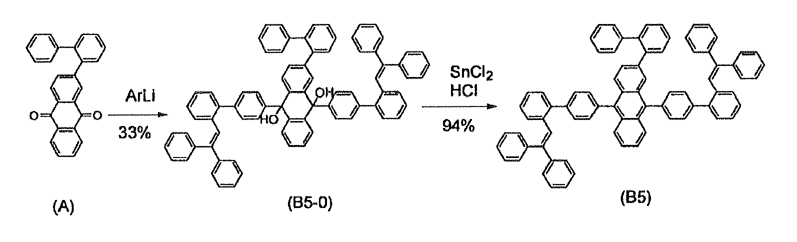

### (1) Synthesis of 2-(2-biphenylyl)-9,10-bis(4-(2-(2,2-diphenylvinyl)phenyl) phenyl)-9,10-dihydroxy-9,10-dihydro anthracene [Compound (B5-0)]

Under an atmospheric argon gas flow, 4-(2,2-diphenylvinyl)phenyl)-1-bromobenzene in an amount of 4.5 g (11 mmol, 2.6 eq) was dissolved into a mixed solvent of anhydrous toluene in an amount of 20 milliliter and anhydrous THF in an amount of 20 milliliter, followed by cooling down to the temperature of ― 20 °C by means of dry ice / methanol bath. Adding hexane solution of n-butyllithium in an amount of 7.6 milliliter (1.59 mol/L, 12 mmol; 1.1 eq), the resultant solution was stirred at the temperature of -20 °C for 1 hour. Into the resultant solution, 2-(2-biphenylyl)-9,10-anthraquinone [Compound (A)] in an amount of 1.5 g (4.2 mmol) was added and after stirring at the room temperature for 9 hours, it was stood alone for a night.
The resultant reaction mixture was deactivated with the use of saturated ammonium chloride aqueous solution in an amount of 50 milliliter, and an organic layer was separated. The organic layer was washed with the use of saturated sodium chloride solution in an amount of 50 milliliter and then, dried with the use of magnesium sulfate, followed by distillation of the solvent and as a result, yellow oil was obtained. The yellow oil was purified in accordance with column chromatography (filled with silicagel). The purification was carried out by eluting with the use of a mixed solvent of hexane and 50 % by mass of dichloromethane, followed by eluting with the use of mixed solvent of dichloromethane and 3 % by mass of methanol. As a result of the purification, 1.4 g of a white amorphous solid compound was obtained (yield: 33 %). The white amorphous solid was identified as the Compound (B5-0) in accordance with ¹H-NMR. The measurement results of ¹H-NMR are shown as the following:
¹H-NMR (CDCl₃, TMS) δ 2.22 (1H, s), 2.76 (1H, s), 6.43 (2H, d, J=8Hz), 6.7-7.5 (49H, m), 7.7-7.9 (3H, m)

### (2) Synthesis of 2-(2-biphenylyl)-9,10-bis(4-(2-(2,2-diphenylvinyl)phenyl)phenyl) anthracene [Compound (B5)]

Suspending 2-(2-biphenylyl)-9,10-bis(4-(2,2-diphenylvinyl)phenyl)phenyl)-9,10-hydroxy-9,10-dihydro anthracene [Compound (B5-0)] in an amount of 1.4 g (1.4 mmol) and stannic chloride dihydrate in an amount of 6.2 g (27 mmol; 20 eq) into 23 milliliter of THF, and adding concentrated hydrochloric acid in an amount of 14 milliliter, the resultant solution was refluxed for 10 hours.
The resultant reaction mixture was separated by filtration, washed with the use of water and methanol successively, followed by drying and as a result, pale yellow solid was obtained. The pale yellow solid was purified in accordance with column chromatography (filled with silicagel). The purification was carried out by eluting with the use of a mixed solvent of hexane and 33 % by mass of dichloromethane. As a result of the purification, 1.3 g of a pale yellow solid compound was obtained (yield: 94 %). The pale yellow solid was identified as the Compound (B5) from the results in accordance with ¹H-NMR and FD-MS measurement, etc. The results of the measurement in accordance with ¹H-NMR and FD-MS are shown as the following:

¹H-NMR (CDCl₃, TMS) δ 6.7-7.8 (54H, m) all-H
FDM S, calcd for C₇₈H₅₄= 990, found m/z =990 (M⁺, 100), 495 (M²⁺, 10)
λ max, 409,385,369 nm (PhMe)
Fmax, 438 nm (PhMe, λ ex =410 nm)
Ip =5.69 eV (100 nanowatt, 21Y/eV)
Eg= 2.88 eV
Tg= 128 °C
Solubility of toluene: 101 mg/milliliter or greater

### [Example 7]

A glass substrate (manufactured by GEOMATEC Company) of 25 mm×75 mm×1.1 mm thickness having an ITO transparent electrode was cleaned by application of ultrasonic wave in isopropyl alcohol for 5 minutes and then by exposure to ozone generated by ultraviolet light for 30 minutes. The glass substrate having the transparent electrode lines which had been cleaned was attached to a substrate holder of a vacuum vapor deposition apparatus. On the surface of the cleaned substrate at the side having the transparent electrode, a film of N,N'-bis(N,N'-diphenyl-4-aminophenyl)N,N'-diephenyl-4,4'-diamino-1,1'-biphenyl (referred to as TPD232, hereinafter) having a thickness of 60 nm was formed so that the formed film covered the transparent electrode. The formed film of TPD232 worked as the hole injecting layer. Successively, a film of N,N',N'-tetra(4-biphenyl)-diamino biphenylene (TBDB film) with a film thickness of 20 nm was formed over the film of TPD232. The formed TBDB film worked as the hole transporting layer. Further, the above Compound (A6) and a following dopant (PAVB) were co-vapor deposited over the BPTPD film at a vapor deposition speed ratio of 40 : 2, resultantly forming a film having a thickness of 40 nm. The formed film worked as a light emitting layer. On the film formed above, a film of tris (hydroxyquinoline) aluminum (Alq below) having a thickness 10 nm was formed. The formed film of Alq worked as an electron injecting layer.

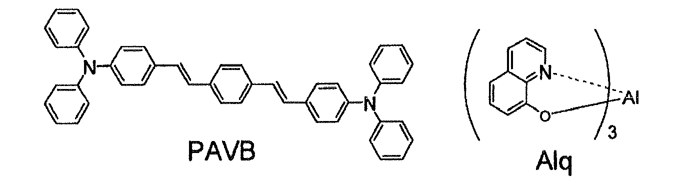

Thereafter, Li (the source of lithium: manufactured by SAES GETTERS Company) as a reductive dopant and Alq were binary vapor deposited and an Alq:Li film (film thickness: 10 nm) was formed as the electron injecting layer (or the cathode). On the Alq:Li film, aluminum was deposited to form a metal cathode and an organic El device was fabricated. Applying DC voltage of 5.6 V across the device, an electric current with density of 1.2 mA/cm² flowed and blue light emission at a luminance of 99 cd/m² was observed. The CIE chromaticity coordinates were (0.15, 0.29). The current efficiency was 8.2 cd/A; 4.7 lm/W. Further, as a result of subjecting the device to a continuous test by feeding a constant electric current starting at an initial luminance of 500 cd/m², it was confirmed that the half lifetime until the luminance reduced to the half value was 2000 hours.

### [Examples 8 to 12]

The devices were fabricated in the same manner as Example 7 except that Compounds shown in Table 1 were employed instead of the Compound (A6) in Example 7. Applying DC voltage across the devices, the current efficiencies and the half lifetimes were measured in the same manner as Example 7. The results are shown in Table 1.

**[Table 1]**

| Examples | Compounds | Current Efficiency (cd/A) | Half Lifetime (hours) |
|---|---|---|---|
| 7 | (A6) | 8.2 | 2000 |
| 8 | (A4) | 8.3 | 1800 |
| 9 | (A5) | 8.1 | 1900 |
| 10 | (A7) | 8.2 | 2300 |
| 11 | (B1) | 7.8 | 1900 |
| 12 | (B5) | 7.9 | 1700 |

### [Example 13]

A glass substrate (manufactured by GEOMATEC Company) of 25 mm×75 mm×1.1 mm thickness having an ITO transparent electrode was cleaned by application of ultrasonic wave in isopropyl alcohol for 5 minutes and then by exposure to ozone generated by ultraviolet light for 30 minutes. On the substrate, a film of polyethylene dihydroxy thiophene / polystyrene sulfonate (PEDOT/PSS) for the use of the hole injecting layer with film thickness of 100 nm was formed in accordance with a spin coat process and then, a solution prepared by dissolving Compound (A6) and the above PAVB into toluene solution of 1 % by mass was applied over PEDOT/PSS in accordance with a spin coat process to form a light emitting layer. The film thickness was 50 nm. On the film formed above, a film of tris(8-quinolinol)aluminum (referred to as Alq film, hereinafter) having a thickness of 10 nm was formed. The film of Alq worked as the electron injecting layer. Thereafter, Li (the source of lithium: manufactured by SAES GETTERS Company) as a reductive dopant and Alq were binary vapor deposited and an Alq:Li film was formed as the electron injecting layer (or the cathode).
On the Alq:Li film, metallic aluminum was vapor deposited to form a metal cathode and an organic El device was prepared. On the Alq:Li film, aluminum was vapor deposited to form a metal cathode and an organic EL device was fabricated. Applying DC voltage of 5.5 V across the device, an electric current with density of 3.5 mA/cm² flowed and blue light emission at a luminance of 1109 cd/m² was observed. The current efficiency was 3.1 cd/A; 1.81 m/W. Further, as a result of subjecting the device to a continuous test by feeding a constant electric current starting at an initial luminance of 100 cd/m², it was confirmed that the half lifetime until the luminance reduced to the half value was 250 hours.

### [Examples 14 to 18]

The devices were fabricated in the same manner as Example 13 except that Compounds shown in Table 1 were employed instead of the Compound (A6) in Example 13. Applying DC voltage across the devices, the current efficiencies and the half lifetimes were measured in the same manner as Example 13. The results are shown in Table 2.

**[Table 2]**

| Examples | Compounds | Current Efficiency (cd/A) | Half Lifetime (hours) |
|---|---|---|---|
| 13 | (A6) | 3.1 | 250 |
| 14 | (A4) | 2.8 | 230 |
| 15 | (A5) | 3.0 | 200 |
| 16 | (A7) | 3.2 | 260 |
| 17 | (B1) | 2.6 | 240 |
| 18 | (B5) | 2.5 | 180 |

### [Comparative Example 1]

Organic EL device was fabricated in the same manner as Example 13 except that Compound (e) below that is described in Japanese Unexamined Patent Application Laid Open No. Hei 11-323323 was employed instead of Compound (A6). Current efficiency of the device was 6.8 cd/A, and the half lifetime starting at an initial luminance of 500 cd/m² was as short as 150 hours.

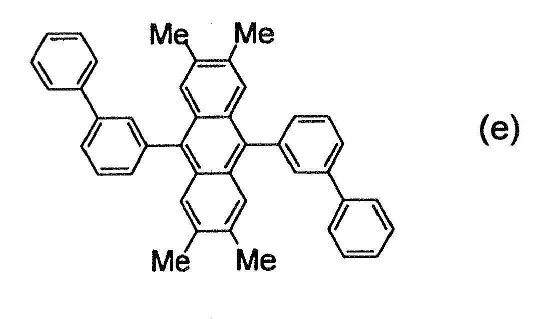

### [Comparative Example 2]

Although a repetition of Example 13 was tried employing Compound (g) below described in Japanese Unexamined Patent Application Laid-Open No. 2001-335516 instead of the Compound (A6), preparing spin coat solution was impossible because a solubility into toluene was extremely small as equal to or lower than 0.1 % by mass.

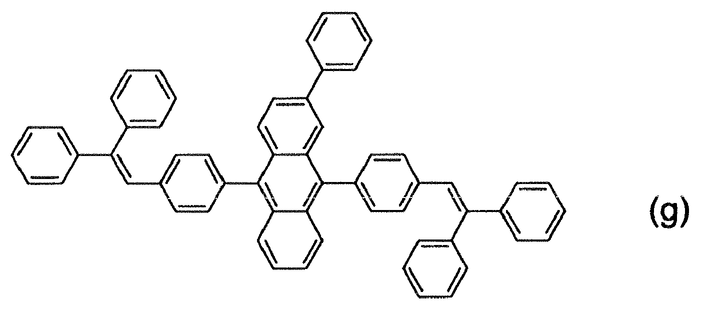

### INDUSTRIAL APPLICABILITY

The organic EL device of the present invention can be used, for example, for a planar light emitting member for a flat panel display of wall televisions, a back light of copiers, printers and liquid crystal displays, a light source for instruments, a display panel and a marking light.

## Claims

1. An aromatic compound represented by a following general formula (1): 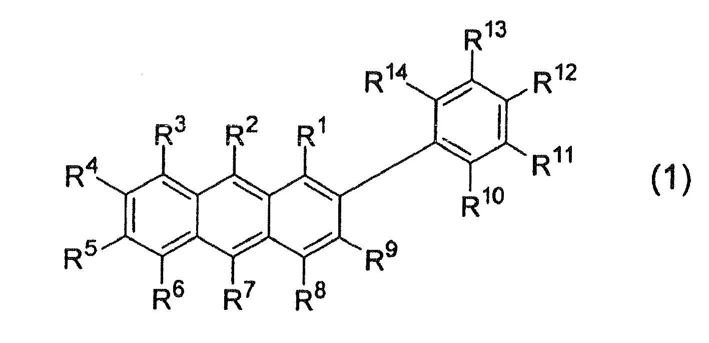 wherein R¹ to R¹⁴ each independently represents any one selected from a group consisting of a hydrogen atom, a halogen atom, a substituted or unsubstituted alkyl group having 1 to 40 carbon atoms, a substituted or unsubstituted alkenyl group having 2 to 40 carbon atoms, a substituted or unsubstituted alkynyl group having 2 to 40 carbon atoms, a substituted or unsubstituted alkoxy group having 1 to 40 carbon atoms, a substituted or unsubstituted aryl group having 6 to 40 carbon atoms, a substituted or unsubstituted heteroaryl group having 3 to 40 carbon atoms;
at least one of R¹ to R⁹ represents a substituted or unsubstituted aryl group having 6 to 40 carbon atoms; and
at least one of R¹⁰ or R¹⁴ represents a substituted or unsubstituted aryl group having 6 to 40 carbon atoms.

2. The aromatic compound according to Claim 1, wherein at least one of R² or R⁷ represents a substituted or unsubstituted aryl group having 6 to 40 carbon atoms.

3. A luminescent organic solution which comprises the aromatic compound according to Claim 1 or Claim 2.

4. A material for an organic electroluminescence device which comprises the aromatic compound according to Claim 1 or Claim 2.

5. An organic electroluminescence device which comprises at least one organic thin film layer comprising a light emitting layer sandwiched between a pair of electrodes consisting of an anode and a cathode, wherein at least one of the organic thin film layer comprises the material for the organic electroluminescence device according to Claim 4.

6. The organic electroluminescence device according to Claim 5, wherein the light emitting layer further comprises an arylamine compound.

7. The organic electroluminescence device according to Claim 5, wherein the light emitting layer further comprises a styrylamine compound.
